# EUROPÄISCHE PATENTSCHRIFT

(11) **EP 2 621 749 B1**
(45) Veröffentlichungstag und Bekanntmachung des Hinweises auf die Patenterteilung: **25.03.2015**
(21) Anmeldenummer: 11764484.9
(22) Anmeldetag: 29.09.2011
(51) Int. Cl.: B60K 28/06, A61B 10/00

(54) **DROGEN-INTERLOCK-SYSTEM MIT SICHERHEITSFUNKTION**
DRUG INTERLOCK SYSTEM HAVING A SAFETY FUNCTION
SYSTÈME ANTIDÉMARRAGE ANTIDROGUE COMPORTANT UNE FONCTION DE SÉCURITÉ

(30) Priorität: 30.09.2010 DE 102010047177
(43) Veröffentlichungstag der Anmeldung: 07.08.2013
(73) Patentinhaber: Dräger Safety AG & Co. KGaA, 23560 Lübeck (DE)
(72) Erfinder: POLZIUS, Rainer, 23566 Lübeck (DE); MORLEY, Stefan, 23556 Lübeck (DE); REINHART, Michael, 23617 Stockelsdorf (DE)
(74) Vertreter: UEXKÜLL & STOLBERG
(86) Internationale Anmeldenummer: PCT/EP2011/004877
(87) Internationale Veröffentlichungsnummer: WO 2012/041505

(56) Entgegenhaltungen:
- WO-A2-2009/083964
- US-A- 6 022 326
- US-A1- 2004 022 687

## Beschreibung

Die vorliegende Erfindung betrifft allgemein eine Vorrichtung, die ausgestaltet ist, um das Starten eines Fahrzeugs, das Bedienen einer Maschine, durch einen unter Drogen stehenden Fahrer bzw. Benutzer zu verhindern. Eine solche Vorrichtung wird nachfolgend als "Drogen-Interlock-System" bezeichnet und verkörpert allgemein die Kombination aus einem Drogenmessgerät sowie einer Wegfahrsperre für ein Kraftfahrzeug, einer Blockiereinrichtung für eine Maschine oder korrespondierend auch eine nur bei Erfüllung bestimmter zulässiger Bedingungen freigegebene Starteinrichtung für eine Maschine oder Betätigungseinrichtung für ein Kraftfahrzeug.

Aus dem Stand der Technik sind sogenannte Alkohol-Interlock-Systeme bekannt, die verwendet werden, um einen alkoholisierten Fahrer nach einer positiven Alkoholmessung daran zu hindern, den Motor eines Fahrzeugs (PKW, LKW, Bus, etc.) zu starten, eine Maschine zu bedienen oder einen gesicherten Bereich in einem Unternehmen zu betreten. Ein solches Alkohol-Interlock-System beinhaltet im Wesentlichen ein Alkoholmessgerät, das sich in der Regel im Inneren des Fahrzeugs befindet, sowie ein mit dem Alkoholmessgerät gekoppeltes Steuergerät, das beispielsweise unter dem Armaturenbrett des Fahrzeugs fest installiert und ausgestaltet ist, um die Stromzufuhr zum Anlasser des Fahrzeugs freizuschalten oder zu blockieren. Das Alkoholmessgerät ist vorzugsweise ein Atemalkoholmessgerät, das als ein Handgerät ausgestaltet und über ein elektrisches Verbindungskabel mit dem Steuergerät verbunden ist.

Diese bekannten Alkohol-Interlock-Systeme sind in großer Anzahl auch in sogenannten Offender-Programmen im Einsatz, bei denen Personen das Führen eines Fahrzeugs nur unter der Auflage gestattet ist, dass sie ein solches System in ihr Fahrzeug einbauen lassen. Diese Auflagen sind im Allgemeinen zeitlich befristet, und die Systeme werden nach Ablauf dieser Befristung wieder aus dem Fahrzeug entfernt. Alkohol-Interlock-Systeme sind somit für den Staat und auch für den beispielsweise wegen Trunkenheit verurteilten Fahrer eine kostengünstige Alternative zu einer eventuellen Haftstrafe oder (in den meisten Fällen) zum Entzug der Fahrerlaubnis. Da die verurteilten Fahrer ein Alkohol-Interlock-System in der Regel nur zeitlich begrenzt einbauen müssen, werden solche Systeme in diesen sogenannten Offender-Programmen zumeist an die Fahrer vermietet. Es fallen also nur die Mietgebühr und evtl. Kosten für die speziellen Mundstücke an.

Ebenso bekannt sind Verfahren zur Bestimmung von Substanzen in Körperflüssigkeiten. Die zugehörigen Nachweisverfahren umfassen chemische, biochemische, elektrochemische und optische Verfahren. So ist beispielsweise die spezifische Reaktion von Cannabinoiden mit dem Gibbs Reagenz bekannt, die entweder direkt durch Farbreaktion oder indirekt durch elektrochemische Detektion erfassbar ist (Studies on Hashish. IV Colour reactions of Cannabinols, Qual. Plant. Mater. Veg. XXII: 7-13, 1972). Beschrieben ist auch die direkte elektrochemische Detektion von Cannabis vorzugsweise nach chromatographischer Auftrennung der Probenbestandteile (Analysis of Cannabinoids, Research Monographs 42, NIDA, 1982). Die optischen Eigenschaften gängiger Drogen werden gemeinsam mit einem Überblick über Messtechniken z.B. in der Arbeit von Özlem Baran, "Determination of narcotic and psychotropic substances by using infrared spectroscopy", Middle East Technical University, July 2005. (http://etd.lib.metu.edu.tr/upload/12606293/index.pdf) dargestellt. Für die Spurenanalytik von Arzneimitteln oder illegalen Drogen aus Körperflüssigkeiten, wie beispielsweise Blut, Urin, Schweiß oder Speichel, werden insbesondere immunchemische Verfahren eingesetzt.

Bei der Sammlung von Körperflüssigkeiten kommt es nicht unwesentlich darauf an, eine ausreichende Menge bzw. ein ausreichendes Volumen an Körperflüssigkeit für die erforderliche Analyse zu sammeln und bereitzustellen. Beispielsweise ist bei einer Blutentnahme die Menge des entnommenen Blutes durch die Skala der Spritze oder bei der Nutzung von Urin durch die Skala am Messgefäß leicht kontrollierbar. Bei auf Speichel basierenden Tests kann der Füllstand z.B. durch einen Farbumschlag des Probennehmers bei Erreichen einer Sollmenge angezeigt werden. Die Entscheidung, ob eine ausreichende Menge an Körperflüssigkeit zur Verfügung steht, wird aber nicht von der zu testenden Person getroffen, sondern von der Person (zum Beispiel einem Arzt), die den Test durchführt. Im Vergleich dazu wird bei Atemalkoholmessgeräten die Volumenmessung durch einen Gasflusssensor im Gerät durchgeführt.

Die obigen Verfahren werden als Schnelltests für die arbeitsmedizinische Überwachung von Personen an gefährlichen Arbeitsplätzen oder auch für die Untersuchung von Autofahrern bei Straßenkontrollen eingesetzt.

Wie im "NHTSA Report DOT HS 811 249 2007 National Roadside Survey of Alcohol and Drug Use by Drivers: Drug Results" dargelegt, ist neben dem Konsum von Alkohol auch der Drogenkonsum beim Führen von Kraftfahrzeugen ein häufig auftretendes Delikt. Daher besteht der grundsätzliche Wunsch, Fahrzeugführer nicht nur auf Alkoholkonsum sondern auch auf Drogenkonsum zu testen und im Falle des Konsums von Drogen das Starten eines Fahrzeugs zu verhindern.

Aus der WO 2009/083964 A2, die den Oberbegriff des Anspruchs 1 bildet, ist ein Alkohol-Interlock-Gerät bekannt, das einen Alkoholtest anhand einer Speichelprobe durchführt. Die Abgabe einer ausreichenden Speichelmenge wird durch eine Person entweder für einen in den Mund gesteckten Probenträger nach Ablauf einer bestimmten Zeit als gegeben angenommen oder anhand des Füllstands einer den Speichel aufnehmenden Kapillare abgelesen. Das Interlock-Gerät kann auch modifiziert werden, um den Speichel auf Drogen zu testen. Dieses Interlock-Gerät hat den Nachteil, dass Messfehler auftreten könnten, wenn eine Messung mit einer nicht ausreichenden Speichelmenge erfolgt, da dies dazu führen könnte, dass eine geringere Alkoholkonzentration gemessen wird als tatsächlich im Körper vorhanden ist.

Grundsätzlich besteht bei allen bekannten Interlock-Systemen auch eine Möglichkeit der Manipulation, indem beispielsweise eine vom System angeforderte Probe von einer zweiten Person abgegeben wird, die nicht die zu überprüfende Person ist. Um eine solche Umgehungsmöglichkeit auszuschließen, weisen bekannte Alkohol-Interlock-Systeme üblicherweise eine Wiederholungsfunktion der Alkoholmessung auf. Das heißt, das Interlock-System fordert den Fahrer nach einem zufällig generierten Zeitintervall erneut auf, eine Alkoholmessung durchzuführen. Auf diese Weise sollen Manipulationen zumindest erschwert werden.

Die DE 197 42 261 A1 beschreibt eine Vorrichtung zum Blockieren der Bedienung eines Fahrzeugs durch einen alkoholisierten Fahrer. Dabei ist das Alkoholmessgerät ausgestaltet, um an einem Körperteil (Arm oder Bein) des Fahrers befestigt zu werden und um den Alkoholpegel des Fahrers mit Hilfe eines elektrochemischen Gassensors über die Hautpermeation messen zu können. Die eigentliche Auswerteeinheit der Vorrichtung, über die eine Freigabe bzw. ein Blockieren des Fahrzeugs erfolgt, ist fest in dem Fahrzeug montiert und kommuniziert drahtlos mit dem Messgerät.

Die US 7,256,700 B1 betrifft ein Interlock-System, mit Hilfe dessen das Starten eines Fahrzeugs durch einen alkoholisierten Fahrer verhindert wird. Das Interlock-System ist mit einem Mobiltelefon oder einer ähnlichen Kommunikationseinrichtung gekoppelt, mit der eine fehlgeschlagene Alkoholmessung kommuniziert wird, indem über das Mobiltelefon eine Sprachmitteilung versendet wird. Ferner können über das Mobiltelefon auch Daten versendet werden, die in dem Interlock-System gespeichert sind.

Die US 2007/0273537 A1 offenbart ein kombiniertes Test- und Lokalisierungssystem, das unter anderem ein Interlock-System beinhaltet. Das Interlock-System dient dabei auf bekannte Weise dazu, das Starten eines Fahrzeugs durch einen alkoholisierten Fahrer zu verhindern. Das System ist ferner mit einem EMHA-System (Electronic Monitoring Home Arrest) ausgestattet, das beispielsweise über ein Mobiltelefon mit einem entfernten Server kommunizieren kann. Über dieses Mobiltelefon können aber auch in dem Interlock-System gespeicherte Daten an den Server übertragen werden.

Die US 2006/0173256 A1 betrifft ein Interlock-System, um das Betreiben eines Fahrzeugs oder einer Maschine durch eine alkoholisierte Person zu unterbinden. Hierzu werden Verfahren und Vorrichtungen zur nicht-invasiven Messung von Alkohol und anderen Substanzen beschrieben.

Die US 6,022,326 betrifft eine Vorrichtung und ein Verfahren zum automatischen oder halb-automatischen Sammeln von Speichel. Die Vorrichtung weist ein Mundstück an einem Rohr auf. Das Rohr ist über eine flexible Leitung mit einem Interface-Abschnitt verbunden. Dadurch wird Speichel über die Atmung in die Vorrichtung transportiert. Die Luft wird entfernt, und Speichel wird in einem Sammelbehälter gesammelt. Zum Sammeln von flüchtigen Komponenten werden die Luftströmung, der Unterdruck, der Durchmesser und die Länge der Leitung und die Sammelzeitpunkte gesteuert und limitiert, um so den Verlust an flüchtigen Komponenten zu reduzieren.

Während der Benutzung des Fahrzeugs können in einem Datenspeicher des Interlock-Systems viele der für den Einsatz relevanten Ereignisse aufgezeichnet werden, wie zum Beispiel Datum, Uhrzeit, Abgabe einer Probe, gemessene Probenwerte, Motorstarts- und Motorstops sowie Manipulationsversuche des Interlock-Systems. Diese Daten können zu einem Protokoll zusammengestellt und zum Beispiel mit Hilfe eines Datenkabels ausgelesen werden. Das Auslesen kann beispielsweise in einer autorisierten Werkstatt oder vor Ort durch einen autorisierten Monteur erfolgen.

Ein Nachteil der bekannten Interlock-Systeme besteht darin, dass sie nicht von einer entfernt gelegenen Zentrale deaktiviert bzw. reaktiviert werden können. Sollte zum Beispiel aufgrund eines technischen Defekts des Interlock-Systems oder aufgrund einer Fehlfunktion des Erfassungssystems das Starten des Fahrzeugs verhindert werden, so ist der Fahrer folglich nicht in der Lage, sein Fahrzeug ohne fremde Hilfe zu starten. Eine Freischaltung bzw. eine Deaktivierung des defekten Interlock-Systems kann in einem solchen Fall nur vor Ort am Interlock-System des Fahrzeugs selbst durchgeführt werden, indem ein autorisierter Monteur zum Beispiel mit Hilfe eines Computers, der über ein Datenkabel mit einer entsprechenden Schnittstelle des Interlock-Systems verbunden wird, einen geheimen Freischalt-Code in das Interlock-System eingibt, wodurch das Interlock-System freigeschaltet bzw. deaktiviert wird und sich der Motor des Fahrzeugs wieder starten lässt. Alternativ muss das Interlock-System in einer Werkstatt repariert und reaktiviert werden.

Es ist Aufgabe der vorliegenden Erfindung, ein vorteilhaftes Interlock-System zur Verfügung zu stellen, mit dem die Konzentration von in einer Körperflüssigkeit enthaltenen Drogen oder anderen Substanzen ermittelt werden kann und das ausgestaltet ist, um bei einem positivem Erfassungsergebnis beispielsweise das Starten eines Kraftfahrzeugs zu verhindern.

Zur Lösung dieser Aufgabe dient ein Interlock-System mit den Merkmalen des Patentanspruchs 1. In den abhängigen Patentansprüchen sind vorteilhafte und bevorzugte Weiterbildungen des erfindungsgemäßen Interlock-Systems angegeben.

Es sei angemerkt, dass das Interlock-System der Erfindung in dieser Beschreibung als "Drogen-Interlock-System" bezeichnet wird. Dieses Interlock-System kann aber zusätzlich ausgestaltet sein, um neben Drogen auch andere unerlaubte Substanzen zu erfassen, wie zum Beispiel Alkohol.

Das erfindungsgemäße Interlock-System für ein Fahrzeug, umfasst:
- einen Probennehmer, der ausgestaltet ist, um von einem zu testenden Probanden eine ausreichende Körperflüssigkeitsprobe aufzunehmen;
- eine mit dem Probennehmer koppelbare Ausleseeinheit zur Erfassung von Substanzen, die in der Körperflüssigkeitsprobe enthalten sind;
- eine mit der Ausleseeinheit koppelbare erste Auswerteeinheit zur Auswertung der durch die Ausleseeinheit erfassten Substanzen, um jeweilige Konzentrationen der erfassten Substanzen zu erhalten, wobei die Erfassung und Auswertung der Substanzen in der Körperflüssigkeit mittels chemischer, immunchemischer, enzymatischer, elektrochemischer oder optischer Nachweismethoden erfolgt,
- eine Messanordnung zum automatischen Messen von Messdaten, die ein Maß dafür sind, welche Menge an Körperflüssigkeit vom Probennehmer aufgenommen wurde, wobei die Messanordnung dazu ausgebildet ist, die Messdaten an eine zweite Auswerteeinheit zu übertragen und wobei die zweite Auswerteeinheit dazu ausgebildet ist, anhand der Messdaten zu ermitteln, ob die vom Probennehmer aufgenommene Menge an Körperflüssigkeit einen vorbestimmten Grenzwert überschreitet; und
- ein mit der ersten Auswerteeinheit und der zweiten Auswerteeinheit koppelbares Steuergerät, das ausgestaltet ist, den Startvorgang des Fahrzeuges zu verhindern, wenn die aufgenommene Menge an Körperflüssigkeit den Grenzwert nicht überschreitet oder wenn mindestens eine der Substanzkonzentrationen oberhalb oder unterhalb eines vorbestimmten Konzentrationsgrenzwerts liegt.

Grundsätzlich funktioniert das Drogen-Interlock-System der vorliegenden Erfindung wie folgt: Nach dem Einschalten der Zündung des Fahrzeugs fordert das Drogen-Interlock-System den Fahrer zur Abgabe einer Probe auf. Das Ergebnis der Mengenbestimmung der aufgenommenen Körperflüssigkeit und die gemessene Drogenkonzentration in dieser Probe entscheiden unabhängig voneinander darüber, ob der Anlasser des Fahrzeugs freigeschaltet wird und der Motor gestartet werden kann. Ein Starten des Motors wird verhindert, wenn zumindest eine der folgenden Situationen vorliegt:
- Die aufgenommene Menge an Körperflüssigkeit überschreitet nicht den vorbestimmten Grenzwert,
- mindestens eine der Substanzkonzentrationen liegt oberhalb oder unterhalb eines vorbestimmten Konzentrationsgrenzwerts.
Das heißt nur wenn keine der beiden Situationen vorliegt, kann der Motor gestartet werden.

Durch das automatische Erfassen der aufgenommenen Menge an Körperflüssigkeit, ist eine die Probenahme überwachende Person, die sicherstellt, dass die abgegebene Flüssigkeitsmenge ausreicht, um ein zuverlässiges Messergebnis der Konzentration der zu erfassenden Substanz zu erhalten, nicht erforderlich. Deshalb kann das erfindungsgemäße Drogen-Interlock-System autark insbesondere in einem Kraftfahrzeug betrieben werden.

Wie vorstehend erläutert, ist das erfindungsgemäße Drogen-Interlock-System ein Drogenmessgerät kombiniert mit einer Wegfahrsperre. Das Ziel des erfindungsgemäßen Drogen-Interlock-Systems besteht grundsätzlich darin, einen unter Drogen stehenden Fahrer daran zu hindern, den Motor des Fahrzeugs zu starten, in dem das Interlock-System eingebaut ist. Alternativ wird der Motor des Fahrzeugs nur für den Startvorgang bzw. Betrieb freigeschaltet, wenn der Drogentest negativ ausfällt, das heißt keine relevante Drogenkonzentration gemessen wird. Durch den Einbau des Interlock-Systems können drogenbedingte Unfälle vermieden werden. Ferner ist das Interlock-System geeignet, langfristige Verhaltensänderungen des Fahrers im Umgang mit Drogen oder anderen Substanzen zu unterstützen.

Vorteilhafterweise ist die Körperflüssigkeit Blut, Urin, Speichel, Tränenflüssigkeit Schweiß oder interstitielle Gewebsflüssigkeit.

Gemäß einer Weiterbildung wird die Temperatur der abgegebenen Körperflüssigkeit während der Probengabe gemessen und mit einem Sollwertbereich verglichen.

Eine von einem Probanden unmittelbar in den Probennehmer abgegebene Körperflüssigkeit hat eine Temperatur, die in einem Sollwertbereich liegt. Eine Körperflüssigkeit, die beispielsweise vor der Einnahme von Drogen abgegeben wurde und längere zeit in einem Behälter aufbewahrt wurde, hat eine Temperatur, die nicht mehr im Sollwertbereich liegt. Dies kann ausgenutzt werden, um eine Analyse der Körperflüssigkeit und somit das Starten des Fahrzeugs davon abhängig zu machen, ob die Temperatur im Sollwertbereich liegt oder nicht. Ein Sollwertbereich kann für jede vom Interlock-System analysierbare Körperflüssigkeit beispielsweise anhand empirischer Daten ermittelt werden.

In einer Ausgestaltung der Erfindung sind die Messdaten ein Maß für einen Farbumschlag einer Detektionszone des Probennehmers, für eine Änderung des Brechungsindex einer Detektionszone des Probennehmers, für elektrochemische Eigenschaften der Körperflüssigkeit oder für die Leitfähigkeit der Körperflüssigkeit.

Eine für eine Analyse ausreichende Menge an Körperflüssigkeit ist dann erfolgt, wenn ein entsprechender Farbumschlag erfolgt ist, eine bestimmte Änderung des Brechungsindex vorliegt oder die elektrochemischen Eigenschaften oder die Leitfähigkeit der Körperflüssigkeit in einem vorgegebenen Parameterbereich liegen oder einen Grenzwert über- oder unterschreiten.

Vorteilhafterweise gehören die Substanzen zu einer Gruppe, die illegalen Drogen wie zum Beispiel Amphetamine, Methamphetamine, Opiate, Kokain, Cannabinoide beinhaltet, oder zu einer Gruppe, die therapeutische Drogen beinhaltet, wie zum Beispiel Benzodiazepine, Methadon, Buprenorphin, trizyklische Antidepressiva.

In einer Weiterbildung wird die Probenahme mittels einer Kamera überwacht, indem optisch festgestellt wird, ob sich ein Mundstück des Probennehmers während der Probenahme im Mund des Fahrers befindet.

Mit Vorteil wird die Probenahme überwacht, indem durch biochemische Nachweisreaktion festgestellt wird, ob es sich bei der Probe um humanen Speichel beziehungsweise um den Speichel des individuellen Fahrers handelt.

Von Vorteil ist, wenn mit Hilfe eines Zufallsgenerators bestimmt wird, zu welchen Zeitpunkten, beispielsweise vor der Fahrt beziehungsweise während der Fahrt, eine Messung erfolgt.

Durch jede der in den letzten drei Absätzen genannten Weiterbildungen wird eine betrügerische Manipulation des Interlock-Systems erschwert. Das heißt, die Wahrscheinlichkeit dafür, dass der Fahrer bei einem negativen Analyseergebnis tatsächlich drogenfrei ist, steigt an, je mehr dieser Maßnahmen gleichzeitig angewandt werden.

Vorteilhafterweise ist der Probennehmer ausgestaltet ist, um in das Steuergerät oder in ein mit dem Steuergerät verbundenes Handgerät eingesetzt zu werden.

Eine Weiterbildung der Erfindung sieht vor, dass das Steuergerät ein mit dem Anlasser des Fahrzeugs gekoppeltes erstes Relais aufweist, das abhängig von der gemessenen Substanzkonzentration geschaltet wird und bei dem das Steuergerät ein zweites Relais aufweist, das parallel zum ersten Relais geschaltet ist und in Reaktion auf drahtlos empfangene Daten geschaltet werden kann.

Bei einer Fehlfunktion des Interlock-Systems können so eine Deaktivierung (ein Freischalten beziehungsweise ein Überbrücken) und/oder eine Reaktivierung des Interlock-Systems anhand von drahtlos empfangenen Daten erfolgen. Hierzu erfolgt beispielsweise zunächst eine beispielsweise telefonische Kontaktaufnahme des Fahrers mit einem Kontroll-System, das je nach Beurteilung der Sachlage die Deaktivierung oder Reaktivierung des Interlock-Systems beispielsweise mittels über ein Mobilfunknetz gesendeter Daten bewirken kann.

Vorteilhafterweise weist die erste Auswerteeinheit und/oder das Steuergerät einen Datenspeicher auf, der mit Hilfe drahtloser Datenübertragung beschrieben und ausgelesen werden kann.

Die im Interlock-System gespeicherten Daten können so drahtlos zu einem entfernt gelegenen Kontroll-System übertragen werden und es können Daten von dem Kontroll-System drahtlos empfangen werden.

Vorteilhafterweise wird durch eine Anzeige auf einem Display und/oder durch ein akustisches Signal angezeigt, wenn die vom Probennehmer aufgenommene Menge an Körperflüssigkeit den vorbestimmten Grenzwert überschreitet.

Auf diese Weise kann der Proband, das heißt der Fahrer des Fahrzeugs, erkennen, wann er die Abgabe der Körperflüssigkeit beenden kann.

Vorzugsweise werden alle relevanten Vorgänge im Zusammenhang mit der Benutzung des erfindungsgemäßen Interlock-Systems in dem Datenspeicher des Interlock-Systems abgelegt. Der Datenspeicher kann zu einem beliebigen Zeitpunkt von dazu berechtigten Personen bzw. Institutionen ausgelesen werden. Das Auslesen des Datenspeichers erfolgt zum Beispiel in regelmäßigen Abständen (z.B. 1x/Monat) in autorisierten Servicewerkstätten. Die Daten werden dann von einer Datenmanagementsoftware verarbeitet, und anschließend werden die relevanten Informationen an eine überwachende Stelle (z.B. Bewährungshelfer) übermittelt (z.B. per Email, SMS, Mobiltelefon oder Fax).

Ein weiterer Vorteil des erfindungsgemäßen Interlock-Systems besteht darin, dass die Weiterfahrt eines Fahrzeugs bei einem technischen Defekt des Interlock-Systems ermöglicht werden kann und/oder dass bestimmte Wartungsaufgaben am Interlock-System durchgeführt werden, ohne dafür eine Servicewerkstatt aufsuchen zu müssen. Wichtig hierbei ist es, dass zur Weiterfahrt bei Defekt des Interlock-Systems die Überbrückung der Interlock-Funktionalität nicht durch den Fahrer allein herbeigeführt werden kann, sondern dass mindestens eine freigebende autorisierte Person oder Institution beteiligt wird. Die Freigabe bzw. Deaktivierung des Interlock-Systems und dessen anschließende Reaktivierung kann hierbei durch eine drahtlose Kommunikation zwischen dem Interlock-System und der autorisierten Person oder Institution erfolgen, die Zugriff auf das Kontroll-System hat. Das Kontroll-System kann eine durch einen Computer zugängliche Datenbank oder einfach ein Mobiltelefon oder Faxgerät sein. Mit Hilfe der vorliegenden Erfindung kann ebenfalls erreicht werden, dass insbesondere die Freischaltfunktionalität im Falle eines defekten Interlock-Systems nicht Bestandteil des (potentiell defekten) Interlock-Systems ist, sondern dass diese Freischaltfunktionalität an das Interlock-System adaptiert wird.

Die vorliegende Erfindung wurde vorstehend allgemein für die Anwendung in einem Kraftfahrzeug beschrieben. Es soll jedoch verstanden werden, dass das erfindungsgemäße Interlock-System gleichermaßen dazu verwendet werden kann, um die Bedienung einer Maschine durch einen alkoholisierten Benutzer zu blockieren. Beispiele solcher Maschine sind große Baumaschinen, Maschinen in industriellen/chemischen Produktionsanlagen, Kraftwerke, etc.

Die vorliegende Erfindung wird nun anhand einiger Ausführungsbeispiele unter Bezugnahme auf die Figuren beschrieben, die verschiedene Ausgestaltungen des erfindungsgemäßen Interlock-Systems zeigen. Obwohl sich die folgende Beschreibung auf ein Interlock-System bezieht, das auf der Erfassung von Drogen basiert, kann das erfindungsgemäße Interlock-System, wie für den Fachmann offensichtlich, auch für die Erfassung bzw. Messung anderer Substanzen ausgestaltet sein, zum Beispiel für die Erfassung des Alkoholpegels des Fahrers.
- Figur 1: zeigt ein erstes Ausführungsbeispiel des erfindungsgemäßen Drogen-Interlock-Systems, bei dem sich erste und zweite Auswerteeinheit in dem Gehäuse des Steuergeräts befinden und das Handgerät nur der Probenahme dient.
- Figur 2: zeigt eine Explosionsansicht eines Probennehmers.
- Figur 3: zeigt eine detaillierte Ansicht eines optischen Detektors zum automatischen Erfassen einer ausreichenden Speichelmenge in dem Probennehmer.
- Figur 4: zeigt ein zweites Ausführungsbeispiel des erfindungsgemäßen Drogen-Interlock-Systems, bei dem sich erste und zweite Auswerteeinheit in dem Handgerät befindet.

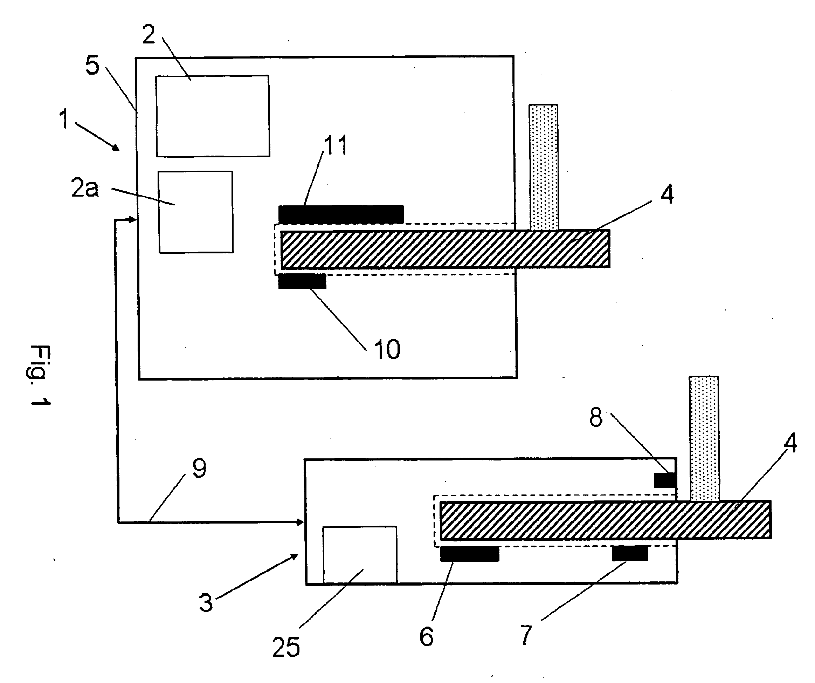

Unter Bezugnahme auf Figur 1 wird nachfolgend ein erstes Ausführungsbeispiel der vorliegenden Erfindung anhand eines Interlock-Systems für ein Kraftfahrzeug beschrieben, bei dem die Auswerteeinheit in dem Gehäuse des Steuergeräts vorgesehen ist und das Handgerät nur zur Aufnahme einer Probe dient. Der Vorteil dieser Ausgestaltung besteht unter anderem darin, dass das Handgerät sehr klein und kompakt sein kann.

Das erfindungsgemäße Drogen-Interlock-System beinhaltet im Wesentlichen ein Steuergerät 1, eine erste Auswerteeinheit 2, eine zweite Auswerteeinheit 2a, ein Handgerät 3 und einen Probennehmer 4. Die erste Auswerteeinheit 2 und die zweite Auswerteeinheit 2a sind im Gehäuse 5 des Steuergeräts 1 untergebracht. Ferner ist bei dieser Ausführung das Handgerät 3 über ein elektrisches Kabel 9 mit dem Steuergerät 1 verbunden. Ebenso sind bei dieser Ausführung die erste Auswerteeinheit 2 und die zweite Auswerteeinheit 2a über digitale Schnittstellen mit den jeweiligen Komponenten des Steuergeräts 1 verbunden.

Selbstverständlich können erste Auswerteeinheit 2 und zweite Auswerteeinheit 2a auch als eine einzige Auswerteeinheit ausgeführt sein, das heißt die Funktionalitäten der ersten Auswerteeinheit 2 und der zweiten Auswerteeinheit 2a werden von einer einzigen Auswerteeinheit bereitgestellt. Ebenso können die erste Auswerteeinheit 2 und/oder die zweite Auswerteeinheit 2a auch im Handgerät 3 angeordnet sein.

Sobald die Person (d.h. der Fahrer bzw. die Fahrerin), die beabsichtigt das Fahrzeug zu führen, die Zündung des Fahrzeugs betätigt (üblicherweise durch Drehen des Zündschlüssels in die Position "Zündung"), wird das Interlock-System in Betrieb genommen. Das System durchläuft dabei zunächst eine eigene Initialisierungs- und Aufwärmprozedur. Sobald das Interlock-System bereit ist, meldet es dem Benutzer beispielsweise durch ein Tonsignal und durch visuelle Ausgabe auf einem Display 25 des Handgeräts 3 den Bereitschaftszustand und fordert den Fahrer gleichzeitig zur Abgabe einer Probe auf.

Selbstverständlich kann das Display auch am Gehäuse des Steuergeräts 1 angeordnet sein. Weiterhin kann die Betriebsbereitschaft des Interlock-Systems und/oder die Aufforderung zur Abgabe einer Probe alternativ oder zusätzlich zur visuellen Anzeige auch mit einem akustischen Signal beispielsweise mittels eines Tones oder einer Tonfolge angezeigt werden.

Vor Abgabe der Körperflüssigkeitsprobe führt der Fahrer nun den Probennehmer 4 in eine entsprechende Öffnung des Handgeräts 3 ein. Dabei liest das Handgerät 3 eine alphanumerische Kontrollsequenz oder eine Kontrollsequenz in Form eines Strichcodes vom Probennehmer 4 ab und überträgt diese über das Kabel 9 an die erste Auswerteeinheit 2 des Steuergeräts 1.

Selbstverständlich kann die Datenübertragung zwischen Handgerät 3 und Steuergerät 1 auch drahtlos erfolgen. In diesem Fall können die Daten beispielsweise per Funk, mittels optischer Signale im sichtbaren oder infraroten Spektralbereich oder auch mittels Ultraschall übertragen werden.

Die Kontrollsequenz ist in dieser Ausführung mit Hilfe eines RFID-Tags (RFID = Radio Frequency Identification) am Probennehmers 4 vorgesehen. Vorzugsweise befindet sich die alphanumerische Kontrollsequenz, der Strichcode bzw. der RFID-Tag an der Unterseite des Probennehmers 4 und wird mit Hilfe eines entsprechenden Sensors 6 ausgelesen, der ein optischer Sensor oder ein RFID-Sensor sein kann, der im Inneren des Handgeräts 3 in Ausrichtung mit der Öffnung für den Probennehmer 4 in einer Position benachbart zum Strichcode bzw. RFID-Tag vorgesehen ist. Wie vorstehend erläutert, können verschiedene Sensor-Typen verwendet werden, und zwar abhängig davon, in welcher Form die Kontrollsequenz am Probennehmer 4 vorliegt.

Nun gibt der Fahrer eine Körperflüssigkeitsprobe (zum Beispiel eine Speichelprobe) in den in das Handgerät 3 eingesetzten Probennehmer 4. Das Handgerät 3 misst dabei die Temperatur der in den Probennehmer 4 eingebrachten Flüssigkeit mit Hilfe eines sich in dem Handgerät 3 befindenden Temperatursensors 7. Die Messdaten des Temperatursensors 7 werden ebenfalls über das Kabel 9 an das Steuergerät 1 übertragen. Im Steuergerät 1 wird beispielsweise durch die erste Auswerteeinheit 2 die gemessene Temperatur mit einem Sollwertbereich verglichen. Liegt der Messwert im Sollwertbereich, wird angenommen, dass es sich um eine ordnungsgemäß abgegebene Probe handelt. Liegt der Messwert außerhalb des Sollwertbereichs, könnte die dem Probennehmer zugeführte Körperflüssigkeit manipuliert worden sein bzw. nicht unmittelbar vom Fahrer abgegeben worden sein. In diesem Fall ist das Steuergerät dazu ausgelegt, den Startvorgang des Fahrzeugs zu verhindern.

Nachdem eine ausreichende Menge an Flüssigkeit auf den Probennehmer 4 aufgebracht bzw. in diesen eingebracht wurde, nimmt der Fahrer den Probennehmer 4 aus dem Handgerät 3 heraus und führt ihn in eine entsprechende Öffnung des Steuergeräts 1 ein, wo mit Hilfe der ersten Auswerteeinheit 2 eine Auswertung der chemischen Bestandteile des Speichels erfolgt.

Zur Prüfung, ob eine ausreichende Menge an Flüssigkeit in den Probennehmer 4 eingebracht wurde, ist in dem Handgerät 3 eine Messanordnung 8 vorgesehen, deren Funktionsweise später erläutert wird. Die von der Messanordnung 8 automatisch während der Probenahme gemessenen Messdaten werden über das Kabel 9 an die zweite Auswerteeinheit 2a übertragen. Stellt die zweite Auswerteeinheit 2a anhand der Messdaten der Messanordnung 8 fest, dass ausreichend Körperflüssigkeit abgegeben wurde, wird dies entweder auf dem Display 25 und/oder durch ein akustisches Signal beispielsweise durch einen Ton oder eine Tonfolge angezeigt.

Als Körperflüssigkeit können neben Speichel selbstverständlich auch Blut, Urin, Tränenflüssigkeit, Schweiß oder interstitielle Gewebsflüssigkeit verwendet werden. Wenn der Probennehmer 4 in das Steuergerät 1 eingesetzt ist, wird mit Hilfe eines Sensors 10, der dem Sensor 6 des Handgeräts 3 entspricht, und unter Steuerung der ersten Auswerteeinheit 2, die Kontrollsequenz bzw. der Strichcode des Probennehmers 4 erfasst und an die erste Auswerteeinheit 2 des Steuergeräts 1 übermittelt. Ein in der ersten Auswerteeinheit 2 des Steuergeräts 1 sich befindender Algorithmus vergleicht die vom Sensor 6 des Handgeräts 3 und die vom Sensor 10 des Steuergeräts 1 übermittelten Kontrollsequenzen (bzw. RFID-Daten oder Strichcodes). Stimmen diese Daten nicht überein, so wird diese Tatsache in einem Speicher des Steuergeräts 1 abgelegt. Stimmen die Daten überein, so wird durch das Steuergerät 1 die Auswertung der sich im Probennehmer 4 befindenden Probe durch die erste Auswerteeinheit 2 eingeleitet. Zur Erfassung der Inhaltsstoffe der Probe im Probennehmer 4 ist an einer geeigneten Position benachbart zur Öffnung in dem Steuergerät 1 eine optische Ausleseeinheit 11 vorgesehen, deren Funktionsweise später erläutert wird.

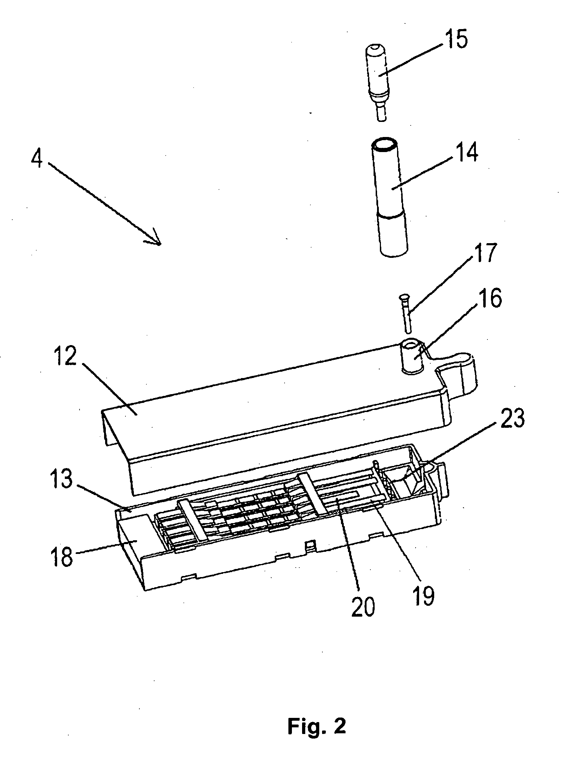

Figur 2 zeigt eine Explosionsansicht von einer bevorzugten Ausführungsform des Probennehmers 4 aus Figur 1. Der Probennehmer weist ein Gehäuse mit einem Oberteil 12 und einem Unterteil 13, eine integrierte Probenaufnahmevorrichtung mit einem Mundstückhalter 14 und einem porösen Mundstück 15, eine in der Probenöffnung 16 des Gehäuses verankerte Reagenzdepot-Komponente 17 sowie auf einer Trägerplatte 18 gelagerte immunchemische Teststreifen 19, 20 auf.

Die integrierte Probenaufnahmevorrichtung (Probennehmer) 4 beinhaltet ein poröses Mundstück 15 zur Aufnahme von Speichel und einen Mundstückhalter 14 als Stützelement für das Mundstück 15 einerseits und als Verbindungselement zur Überführung der Probe in das Gehäuseinnere des Probennehmers 4. Durch Kontaktbeprobung mit der Mundschleimhaut wird die Speichelprobe in das poröse Mundstück 15 aufgenommen und dabei vorzugsweise durch Kapillarkräfte aufgesaugt.

Es ist im Allgemeinen notwendig, ein ausreichendes Speichelvolumen aufzunehmen. Dies wird bei assistierten Drogentests z.B. durch einen von der testenden Person visuell kontrollierbaren Farbumschlag in einem spezifizierten Bereich des porösen Mundstücks gewährleistet.

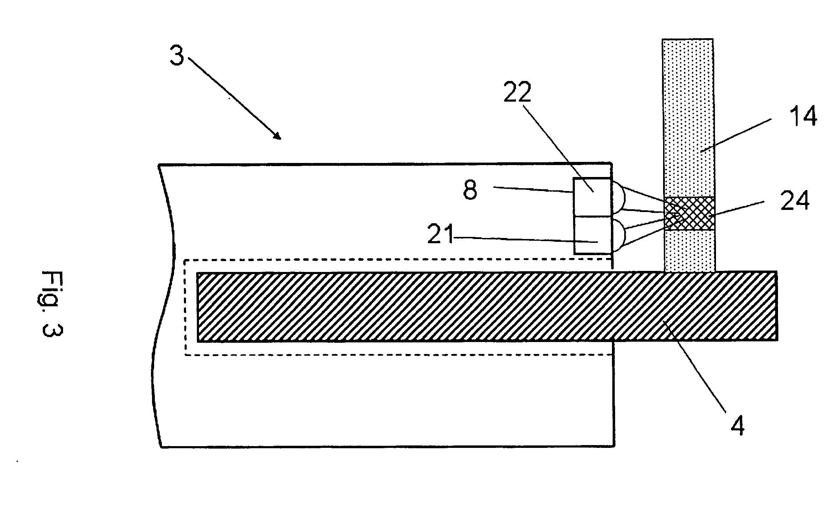

In Figur 3 ist eine Ausgestaltung eines erfindungsgemäßen Auslesesystems dargestellt, das eine automatische Speichelvolumenkontrolle während der Probenahme und somit eine ununterbrochene Probenabgabe ermöglicht. Es handelt sich beispielhaft um ein optisches Ausleseverfahren, bei dem mittels der in Figur 1 dargestellten Messanordnung 8, die Veränderung der optischen Eigenschaften eines als Detektionszone 24 ausgeführten Bereichs des Mundstücks 15 mit Hilfe von sichtbarem Licht ermittelt wird. Hierzu weist die im Handgerät 3 angeordnete Messanordnung 8 eine Licht emittierende LED 21 und einen lichtsensitiven Sensor 22 auf. Es ist bei diesem Ausführungsbeispiel unerheblich, ob die optischen Eigenschaften der Detektionszone 24 in Ausprägung eines Farbumschlages oder mittels Änderung der Reflexionseigenschaften (Brechungsindexänderung ohne Farbumschlag) verändert werden. Handelt es sich um einen Farbumschlag, so wird das von der LED 21 emittierte Licht am Mundstück 15 reflektiert und vom lichtsensitiven Sensor 22 detektiert. Bei Farbumschlag wird eine Intensitätsänderung gemessen, deren Ausmaß (relative Intensitätsänderung) zur Beurteilung des Speichelvolumens benutzt wird. Überschreitet die Intensitätsänderung einen vorbestimmten Grenzwert, ist durch Abgabe einer ausreichenden Menge an Speichel ein entsprechender Farbumschlag bewirkt worden. Die Messdaten der Messanordnung 8, das heißt in diesem Ausführungsbeispiel die gemessene Intensitätsänderung, werden wie zuvor beschrieben an die zweite Auswerteeinheit 2a zur weiteren Verarbeitung übertragen.

Alternativ kann die Messanordnung 8 auch so ausgeführt sein, dass die Kontrolle der Probenmenge mit anderen physikalischen oder chemischen Messverfahren wie beispielsweise der Messung elektrochemischer Größen oder der Leitfähigkeit der Körperflüssigkeit erfolgt.

Insbesondere zum Messen elektrochemischer Größen oder der Leitfähigkeit kann die Messanordnung 8 auch im Probennehmer 4 angeordnet werden. Bei einem Anordnen im Probennehmer können die Messdaten beispielsweise über eine entsprechende elektrische Kontaktierung vom Probennehmer 4 an das Handgerät 3 übertragen werden. Ebenso kann eine Messdatenübertragung vom Probennehmer 4 zum Handgerät 3 drahtlos erfolgen.

Zusätzlich zum Volumen der Probe können Identitätsmerkmale der Probe im System bestimmt werden, um die Manipulationssicherheit des Verfahrens zu steigern. So kann beispielsweise die immunchemische Bestimmung von humanen Serumproteinen sowohl Aufschluss über die Probenmengen als auch über die Herkunft der Probe geben. Anhand der chemischen Zusammensetzung der in der Probe enthaltenen Proteine kann festgestellt werden, ob es sich um humanen Speichel handelt. Eine individualisierte Zuordnung der Probe zu einer bestimmten Person ist beispielsweise über ein molekulargenetisches Profil der in der Körperflüssigkeitsprobe enthaltenen DNA oder RNA möglich. Das poröse Mundstück 15 kann beispielsweise aus Schaumstoffen, gepressten oder geklebten Fasern, oder gesinterten Kunststoffen, Metallen oder Keramiken bestehen.

Die im Mundstück aufgenommene Speichelprobe wird entweder passiv oder aktiv mit Hilfe eines Aktuators in den Innenraum des Gehäuses des Probennehmers 4 überführt (siehe Figur 2). Dies geschieht vorzugsweise durch Erzeugen eines Überdruckes mit einer eindringenden Reagenzflüssigkeit, die, wie in der DE 103 28 984 B4 beschrieben, ein Extrakt oder Filtrat erzeugt, das einen Teil der Probenflüssigkeit enthält.

Im Unterteil 13 des Gehäuses des Probennehmers 4 befindet sich eine Probenwanne 23 zum Vorhalten und Temperieren der flüssigen Probe. Die Probenwanne 23 lässt sich beispielsweise von außen über den Formschluss mit einem Temperierelement aufheizen oder abkühlen.

Die Trägerplatte 18 umfasst mehrere Aufnahmen für immunchromatographische Teststreifen 19, 20, die gegeneinander abgegrenzt sind, um die gegenseitige Beeinflussung ("crosstalk") der kapillaraktiven Nachweiselemente untereinander zu verhindern. Die immunchromatographischen Teststreifen 19, 20 bestehen im Allgemeinen aus kapillaraktiven Trägermaterialien oder einem Verbund verschiedener kapillaraktiver Trägermaterialien oder mikrofluidischen Kanälen, die einen Fluidtransport autonom ermöglichen, nachdem Fluidkontakt mit der flüssigen Probe hergestellt wurde. Vorzugsweise handelt es sich dabei um poröse Schichten von Polymeren oder verklebten und gepressten Fasern, die Depotzonen oder Detektionszonen aufweisen. Speziell bestehen die kapillaraktiven Nachweiselemente aus einem Teststreifenmaterial.

Vorzugsweise werden die kapillaraktiven Nachweiselemente teilweise auf der Trägerplatte fixiert, während ein anderer Teil der kapillaraktiven Nachweiselemente in die Testkassette hineinragt, um in Fluidkontakt mit der flüssigen Probe zu treten.

Nach erfolgtem Fluidkontakt saugen sich die kapillaraktiven Nachweiselemente mit Probenflüssigkeit voll. Infolge der Fluidströmung durch die Teststreifenmaterialien können weitere Reagenzien für die Nachweisreaktion der Analyten solubilisiert werden. Es kommt zur Reaktion oder Komplexbildung mit dem oder den Analyten, die weiter stromaufwärts in einer oder mehreren räumlich abgegrenzten Detektionszonen mittels immunchemischer Interaktion selektiv abgefangen werden. Die Signale in den Detektionszonen können, je nach verwendetem Marker, optisch, magnetisch oder elektrisch ausgelesen werden.

Nach der Auswertung der in diesem Ausführungsbeispiel verwendeten Speichelprobe wird bei einem negativen Testergebnis, wenn keine Zielsubstanz nachgewiesen wurde oder wenn die Konzentration der Zielsubstanz unterhalb eines eingestellte Konzentrationsgrenzwertes liegt, im Allgemeinen das Testergebnis mit einer Mitteilung wie "Test OK" oder "Test NICHT OK" angezeigt. In einer Ausführungsform wird auch die gemessene Konzentration angezeigt. In den Fällen, in denen das Testergebnis positiv ist, also Zielsubstanzen nachgewiesen wurden, wird der weitere Startvorgang des Fahrzeugs nicht freigegeben. In einer Ausführungsform geschieht dies durch Unterbrechung der Zuleitung vom Zündschloss zum Anlasserrelais und in einer anderen möglichen Ausführung durch digitale Interaktion des Steuergerätes mit dem elektronischen Bussystem des Fahrzeugs. In letzterem Fall wird z.B. über den CAN- oder LIN-Bus (CAN = Control Area Network; LIN = Local Interconnect Network) des Fahrzeugs eine Meldung abgesetzt, die von der Recheneinheit des Fahrzeugs als Befehl, das Fahrzeug nicht anzulassen, interpretiert wird.

Nach Abschluss des Messvorgangs wird der Probennehmer 4 wieder entnommen und kann zum Zwecke einer späteren Laborbestätigungsanalyse aufbewahrt werden. Der Probennehmer 4 enthält geeignete Strukturen, wie beispielsweise Kavitäten oder saugfähige Materialien, die einen Teil der überschüssigen Probenflüssigkeit über einen Zeitraum von Tagen bis zu einigen Monaten asservieren. Dies erlaubt eine spätere gerichtsverwertbare Laboranalyse, die entweder über die Identifizierung des Probennehmers 4 oder der individuellen Probe (beispielsweise über DNA-Profile) dem Fahrer des Fahrzeugs retrospektiv zugeordnet werden kann.

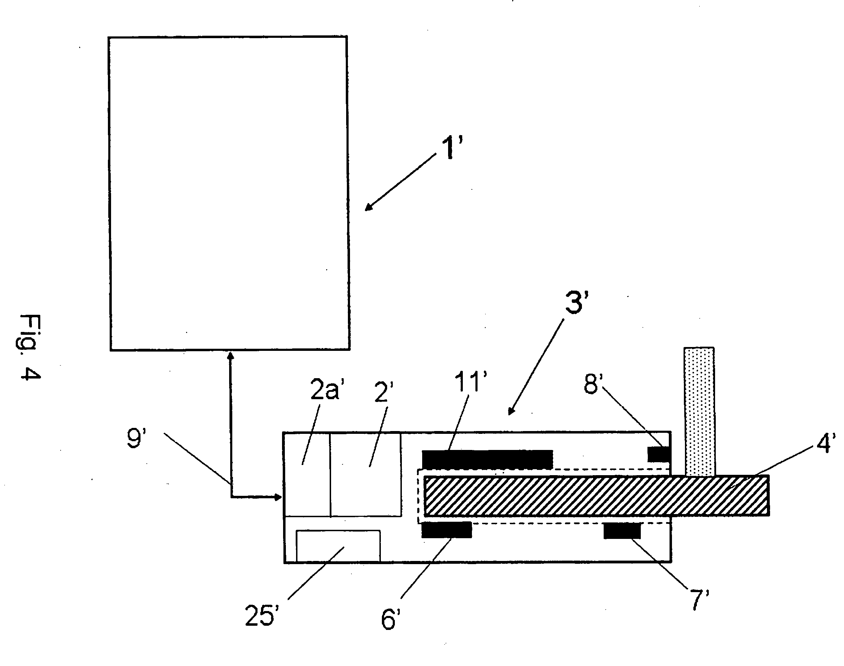

Figur 4 zeigt eine zweite Ausführung der vorliegenden Erfindung, die ebenfalls ein Interlock-System für Kraftfahrzeuge betrifft, bei dem allerdings die erste Auswerteeinheit 2' und die zweite Auswerteeinheit 2a' in einem Gehäuse des Handgeräts 3' angeordnet und als eine bauliche Einheit ausgebildet sind. Selbstverständlich können die Auswerteeinheiten 2' und 2a' auch als separate Einheiten ausgebildet und angeordnet werden. Das Handgerät 3' bildet in dieser Ausgestaltung der Erfindung einen vollständigen Substanzdetektor, mit Hilfe dessen die Probenahme und Analyse erfolgt und der das mit ihm erzielte Messergebnis an das Steuergerät 1' weiterleitet.

Das Drogen-Interlock-System aus Figur 4 weist im Wesentlichen ein Steuergerät 1', ein in der Hand haltbares Auswertegerät, das als Handgerät 3' bezeichnet wird, und einen Probennehmer 4' auf. Auch bei dieser Ausführung ist das Handgerät 3' mit Hilfe eines Kabels 9' mit dem Steuergerät 1' verbunden. Selbstverständlich kann die Verbindung zwischen Handgerät 3' und Steuergerät 1' wie oben im Zusammenhang mit Figur 1 ausgeführt auch drahtlos erfolgen. Sobald die Person, die beabsichtigt, das Fahrzeug zu führen (d.h. der Fahrer), das Zündschloss betätigt, wird das Interlock-System in Betrieb genommen. Es durchläuft dabei zunächst eine eigene Initialisierungs- und Aufwärmprozedur.

Sobald das Interlock-System bereit ist, meldet es dem Fahrer durch ein Tonsignal und/oder eine Anzeige auf einem Display 25' des Handgerätes 3' die Betriebsbereitschaft und fordert dabei gleichzeitig zur Abgabe einer Probe auf. Vor Abgabe der Körperflüssigkeitsprobe führt der Fahrer nun den Probennehmer 4' in das Handgerät 3' ein. Dabei liest das Handgerät 3' eine Kontrollsequenz vom Probennehmer 4' ab und sendet sie über das Kabel 9' an das Steuergerät 1'. Diese Kontrollsequenz ist auch bei dieser Ausführung, wie vorstehend unter Bezugnahme auf Figur 1 beschrieben, beispielsweise mittels eines RFID-Tags am Probennehmer 4' angebracht und verifiziert den jeweiligen Probennehmer mit Hilfe eines Sensors 6' als gültigen Probennehmer. Statt des RFID-Tags können auch andere Informationsträger und entsprechende Sensoren 6' verwendet werden. Nun gibt der Fahrer eine Körperflüssigkeitsprobe (zum Beispiel Speichel) in den Probennehmer. Das Handgerät 3' misst dabei die Temperatur der in den Probennehmer eingebrachten Flüssigkeit mit Hilfe eines Temperatursensors 7'. Im Handgerät 3' wird beispielsweise durch die erste Auswerteeinheit 2' die gemessene Temperatur mit einem Sollwertbereich verglichen. Liegt der Messwert im Sollwertbereich, wird angenommen, dass es sich um eine ordnungsgemäß abgegebene Probe handelt. Liegt der Messwert außerhalb des Sollwertbereichs, könnte die dem Probennehmer zugeführte Körperflüssigkeit manipuliert worden sein beziehungsweise nicht unmittelbar vom Fahrer abgegeben worden sein. In diesem Fall ist das Steuergerät 1' dazu ausgelegt, den Startvorgang des Fahrzeugs zu verhindern.

Mittels einer Messanordnung 8', die beispielsweise so ausgeführt ist wie die Messanordnung 8 gemäß Figur 3, werden automatisch während der Probenahme Messdaten gemessen, die ein Maß dafür sind, welche Menge an Körperflüssigkeit vom Probennehmer aufgenommen wurde. Anhand der Messdaten kann somit überprüft werden, ob die vom Probennehmer 4' aufgenommene Menge an Körperflüssigkeit einen vorbestimmten Grenzwert überschreitet. Die Messdaten werden von der Messanordnung 8' an die zweite Auswerteeinheit 2a' entweder über eine Leitung, beispielsweise eine Leiterbahn oder ein Kabel, oder drahtlos, beispielsweise per Funk, mittels optischer Signale im sichtbaren oder infraroten Spektralbereich oder auch mittels Ultraschall, übertragen und von der zweiten Auswerteeinheit 2a' ausgewertet. Stellt die zweite Auswerteeinheit 2a' fest, dass die vom Probennehmer 4' aufgenommene Menge an Körperflüssigkeit einen vorbestimmten Grenzwert überschreitet, beginnt das Handgerät 3' mit der Auswertung der Probe. Der Beginn der Auswertung der Probe und/oder das Erreichen einer ausreichenden Menge an Körperflüssigkeit kann entweder auf dem Display 25' und/oder durch ein akustisches Signal beispielsweise durch ein Ton oder eine Tonfolge angezeigt werden.

Der Probennehmer 4' hat die gleiche Konfiguration wie Probennehmer 4, der unter Bezugnahme auf Figur 2 und die zugehörige Beschreibung erläutert wurde.

Eine deutliche Verbesserung der Manipulationssicherheit wird weiterhin erreicht, indem der Vorgang der Probengabe durch eine Kamera kontrolliert wird. Dazu wird ein Kameramodul so im Fahrzeug befestigt, dass mindestens der Bereich des Fahrersitzes im Gesichtfeld der Kamera liegt. Dadurch wird es möglich, den Probengeber (Fahrer des Fahrzeugs) zum Zeitpunkt der Probengabe bildlich festzuhalten, sodass erkannt wird, ob sich ein Mundstück des Probennehmers 4, 4' während der Probengabe im Mund des Fahrers befindet. Es ist ferner möglich, dass der Zeitpunkt der Probengabe eindeutig durch die Zufuhr eines Teilstroms der Atemprobe auf einen elektrochemischen Sensor mittels beispielsweise eines durch einen Hubmagneten betätigten Faltenbalges gekennzeichnet ist. Diese Ansaugung stellt einen sehr kurzen Zeitraum von etwa einer Sekunde dar und definiert den Moment der Probengabe ausreichend präzise. Bei der Sammlung von Körperflüssigkeit, insbesondere von Speichel, ist der Sammelvorgang deutlich verlängert, wobei von einer Zeit bis in den Minutenbereich auszugehen ist. Hier zeigt es sich nun, dass es von Vorteil ist, mindestens drei Bilder des Probanden (Fahrer bzw. Fahrerin) aufzunehmen und die Aufnahmezeitpunkte mit der durch die Körperflüssigkeit hervorgerufenen Veränderung der in dieser Ausführung optischen Eigenschaften der Detektionszone zu korrelieren. Es zeigt sich weiterhin, dass es vorteilhaft ist, diese Bilder zu bestimmten Zeitpunkten aufzunehmen, d.h. das erste Bild zu Beginn des Farbumschlags des Mundstücks 15, das zweite Bild in der Mitte des Farbumschlags und das dritte Bild am Ende oder gegen Ende des Farbumschlags. Technisch wird dies dadurch erzielt, dass beginnend mit der Probengabe permanent Bilder in definierten Abständen aufgenommen werden, jedoch nur diejenigen Bilder gespeichert werden, die mit den oben beschriebenen Zeitpunkten des Farbumschlags korrelieren. Durch dieses Verfahren ist sichergestellt, dass nicht eine zweite Person die Speichelprobe geben kann und dann z.B. einen nur beinahe vollständig gefüllten Probennehmer an den Fahrer übergeben kann.

Eine Maßnahme, um die Kosten des Betriebes eines erfindungsgemäßen Drogen-Interlock-Systems für den Benutzer in einem vernünftigen Maß zu halten und um gleichzeitig ein Höchstmaß an Sicherheit für die anweisende Organisation (Unternehmen, Staat) zu gewährleisten, ist die Steuerung der Anforderung von Körperflüssigkeitsproben in Kombination mit einem Zufallsgenerator. Der Zufallsgenerator kann in der Steuereinheit angeordnet sein oder in einem beispielsweise mittels einer Funkverbindung mit der Steuereinheit verbundenen bzw. verbindbaren Rechnersystem einer Überwachungsorganisation.

Da dem zu überprüfenden Probanden (Fahrer) nicht bekannt ist, ob er nur bei einem bestimmten Startversuch überprüft wird, muss er sich bei jedem Startversuch und zusätzlich auch während der Fahrt regelkonform verhalten, da neben einem Ersttest auch ein Wiederholungstest mit einem solchen Zufallsgenerator gekoppelt sein kann. Dies macht ein Mitführen einer präparierten Speichelprobe einer dritten Person im Mund des Probanden höchst ineffizient. Um die Sicherheit weiter zu erhöhen, kann ein Zufallsgenerator auch so ausgelegt sein, dass die Wahrscheinlichkeit eines Tests abends und nachts häufiger ist als morgens.

Eine weitere Ausführungsform ist die Dauerüberwachung im "Home Arrest" Umfeld. Hier gelten die analogen technischen Anforderungen an das Drogen-Messsystem.

Wie eingangs erläutert, kann das Handgerät 3, 3' ein Display 25, 25' sowie mehrere Bedientasten aufweisen. Möchte ein Fahrer sein mit einem Interlock-System ausgestattetes Fahrzeug starten, so muss er zunächst mit Hilfe des Zündschlüssels die Zündung einschalten. Daraufhin erscheint beispielsweise auf dem Display 25, 25' des Handgeräts 3, 3' (zusätzlich kann ein akustisches Signal ertönen) die Aufforderung, eine Speichelprobe in das Mundstück 15, 15' des Handgeräts 3, 3' zu geben. Mit Hilfe des Sensors 11, 11' und der ersten Auswerteeinheit 2, 2' wird nun die Drogenkonzentration gemessen, wenn die zweite Auswerteeinheit 2a, 2a' anhand der Messdaten feststellt, dass die vom Probennehmer aufgenommene Menge an Speichel einen vorbestimmten Grenzwert übersteigt und somit ausreichend Speichel für eine Analyse vorhanden ist. Liegt die Drogenkonzentration innerhalb des definierten Grenzbereichs, dann erfolgt die Freigabe, was durch eine Nachricht auf dem Display 25, 25' des Handgeräts 3, 3' angezeigt wird (auch hier kann zusätzlich ein akustisches Signal ertönen). Anschließend kann der Fahrer mit Hilfe des Zündschlüssels den Motor starten. Bei der Messung der Drogenkonzentration erzeugt die erste Auswerteeinheit 2, 2' ein Signal, das zum Steuergerät 1,1' übertragen wird. Liegt die gemessene Drogenkonzentration innerhalb der definierten Grenzen, so generiert die erste Auswerteeinheit 2, 2' ein entsprechendes Steuersignal zum "Einschalten" eines Relais im Zündschaltkreis zwischen Batterie und Anlasser. Liegt die gemessene Drogenkonzentration außerhalb dieser Grenzen, so bleibt das Relais ausgeschaltet, wodurch ein Starten des Motors verhindert wird. Alternative Steuerkonzepte sind ebenfalls möglich, wie zum Beispiel ein Eingriff auf das Bussystem des Kraftfahrzeugs, wie vorstehend erläutert. Der definierte Grenzbereich, innerhalb dessen ein Starten des Motors zulässig ist, ist in einem Datenspeicher im Steuergerät 1, 1' selbst gespeichert und kann durch eine autorisierte Person bzw. durch eine autorisierte Institution fest eingestellt und natürlich auch verändert werden. Ferner werden alle relevanten Daten, d.h. Zeitpunkt des Startversuchs, gemessene Drogenkonzentration, mögliche Manipulationsversuche, etc., ebenfalls in einem Datenspeicher des Steuergeräts 1, 1' oder der ersten Auswerteeinheit 2, 2' gespeichert und können durch die autorisierte Person bzw. Institution ausgelesen werden. Das Eingeben bzw. Auslesen von Daten kann mit Hilfe eines Datenkabels, das am Steuergerät oder am Handgerät angeschlossen wird, oder auch drahtlos erfolgen. Hierzu kann das Steuergerät alle wesentlichen Komponenten für eine kabellose Datenübertragung von/zu einem nicht dargestellten, entfernt gelegenen Kontrollsystem (zum Beispiel Computer, Server oder Mobiltelefon einer autorisierten Person bzw. Institution) sowie beispielsweise ein Sicherheitsrelais enthalten, um die Funktion des Steuergeräts 1, 1' des Interlock-Systems im Falle einer technischen Störung zu überbrücken.

Die Komponenten zur kabellosen Datenübertragung (zum Beispiel über UMTS, GSM, GPRS, etc.) sind in dem Steuergerät enthalten und ermöglichen das Auslesen eines Datenspeichers im Steuergerät 1, 1' bzw. in der Auswerteeinheit 2, 2', ohne dass hierzu eine Servicewerkstatt aufgesucht werden muss. Die zur Datenübertragung erforderlichen Kommunikationsprotokolle hängen von der verwendeten Art der Kommunikation (UMTS, GSM, GPRS, etc.) ab und sind dem Fachmann bekannt. Die Datenübertragung kann in regelmäßigen Abständen erfolgen, durch ein bestimmtes Ereignis (z.B. ein nicht bestandener Drogentest, eine bestimmte Anzahl von Drogentests oberhalb eines bestimmten Grenzwertes, ein vom Interlock registrierter Manipulationsversuch, der Ablauf einer Serviceoder Kalibrierperiode, etc.) ausgelöst werden oder mit einem Signal (z.B. per SMS oder durch ein anderes geeignetes Protokoll) an das Interlock-System ausgelöst werden.

Die Daten werden dabei vom Datenspeicher des Interlock-Systems an ein Zielsystem (Kontroll-System, Datenmanagement-System, etc.) übergeben. In diesem Zielsystem werden die Daten gespeichert und ggf. verarbeitet. Unabhängig von der Übertragung der Daten aus dem Datenspeicher kann im Fall eines Fehlverhaltens des Fahrers (ein nicht bestandener Drogentest, Manipulationsversuche, etc.) eine Benachrichtigung vom Interlock-System direkt an die verantwortliche Aufsichtspersonen (bzw. an die verantwortliche Institution) geschickt werden (z.B. an ein Mobiltelefon oder per Email/Fax).

Bei einem Defekt des Interlock-Systems kann die Situation auftreten, dass der Fahrer nicht mehr in der Lage ist, den Motor seines Fahrzeugs zu starten. Dieser Zustand kann mit einem Sicherheitsrelais aufgehoben werden, das im Steuergerät vorgesehen und der eigentlichen Funktionalität des Interlock-Systems parallel geschaltet ist. Durch eine solche ODER-Schaltung zwischen dem Sicherheitsrelais und der Funktion des Interlock-Systems wird es ermöglicht, das blockierende Interlock-System zu umgehen, indem das Sicherheitsrelais geschlossen wird. Das Auslösen des Sicherheitsrelais erfolgt dabei mittels drahtloser Datenübertragung zwischen den Kommunikationskomponenten des Steuergeräts und dem entfernt gelegenen Kontroll-System. Dabei sind zwei bevorzugte Ausführungen denkbar. In einer ersten Ausführung verfügt das Steuergerät über einen Sender/Empfänger (Transceiver) in Gestalt eines Mobiltelefon-Submoduls. Das Steuergerät ist somit in der Lage, beispielsweise eine SMS (Short Message Service) zu empfangen und den Absender dieser SMS sowie den Inhalt der SMS zu überprüfen. Der Fahrer des Fahrzeugs muss sich bei dieser Ausführungsform bei einer zur Freischaltung autorisierten Person/Instanz melden. Diese Instanz verschickt nun zum Beispiel über ein vorher benanntes Mobiltelefon eine SMS mit einem Freigabecode an das Mobiltelefon-Submodul des Interlock-Moduls. Die in dem Modul gespeicherte und ausführbare Software überprüft dann beispielsweise, ob der Freigabecode und das absendende Mobiltelefon autorisiert sind. Hierzu werden Verfahren verwendet, die dem Fachmann bekannt sind und hier nicht weiter erläutert werden. Das Modul überbrückt anschließend bei erkannter und bestätigter Autorisierung des Freigabecodes mittels des Sicherheitsrelais die Unterbrechung des Anlasser-Schaltkreises, indem das Sicherheitsrelais geschlossen wird.

In einer anderen bevorzugten Ausführung wird der Freigabecode mittels GPRS (General Packet Radio Service) übermittelt. Bei der Verwendung von GPRS ist die Menge der in einem Paket übermittelten Information potentiell größer, und daher können nicht nur ein praktisch beliebig langer Freigabecode sondern darüber hinaus weitere Informationen übermittelt werden. Diese Informationen umfassen unter anderem die zeitliche Befristung der Überbrückung.

Durch die drahtlose Datenübertragung wird ferner ermöglicht, eine "Fernwartung" durchführen zu können, d.h. Selbsttests des Interlock-Systems oder das Zurücksetzen von Timern oder Sperren (z.B. falls das Interlock nach Verstößen in einen "Lockout-Modus" versetzt wurde, der ein erneutes Starten des Fahrzeugs nicht mehr zulässt). Darüber hinaus können Servicedaten im Interlock-Datenspeicher sowie Parametereinstellungen (z.B. Grenzwerte, etc.) ausgelesen und überschrieben sowie Updates der Gerätesoftware durchgeführt werden.

### BEZUGSZEICHENLISTE

- 1, 1': Steuergerät
- 2, 2': Erste Auswerteeinheit
- 2a, 2a': Zweite Auswerteeinheit
- 3, 3': Handgerät
- 4, 4': Probennehmer
- 5: Gehäuse
- 6, 6': RFID-Sensor
- 7, 7': Temperatursensor
- 8, 8': Messanordnung
- 9, 9': Kabel
- 10: RFID-Sensor
- 11, 11': optische Ausleseeinheit
- 12: Oberteil
- 13: Unterteil
- 14: Mundstückhalter
- 15: Mundstück
- 16: Probenöffnung
- 17: Reagenzdepot-Komponente
- 18: Trägerplatte
- 19, 20: Teststreifen
- 21: LED
- 22: lichtsensitiver Sensor
- 23: Probenwanne
- 24: Detektionszone
- 25, 25': Display

## Patentansprüche

1. Interlock-System für ein Fahrzeug, mit:
- einem Probennehmer (4; 4'), der ausgestaltet ist, um von einem zu testenden Probanden eine ausreichende Körperflüssigkeitsprobe aufzunehmen;
- einer mit dem Probennehmer (4; 4') koppelbaren Ausleseeinheit (11; 11') zur Erfassung von Substanzen, die in der Körperflüssigkeitsprobe enthalten sind;
- einer mit der Ausleseeinheit koppelbaren ersten Auswerteeinheit (2; 2') zur Auswertung der durch die Ausleseeinheit (11; 11') erfassten Substanzen, um jeweilige Konzentrationen der erfassten Substanzen zu erhalten;
- wobei die Erfassung und Auswertung der Substanzen in der Körperflüssigkeit mittels chemischer, immunchemischer, enzymatischer, elektrochemischer oder optischer Nachweismethoden erfolgt, gekennzeichnet durch
- eine Messanordnung (8, 8') zum automatischen Messen von Messdaten, die ein Maß dafür sind, welche Menge an Körperflüssigkeit vom Probennehmer aufgenommen wurde, wobei die Messanordnung (8, 8') dazu ausgebildet ist, die Messdaten an eine zweite Auswerteeinheit (2, 2') zu übertragen und wobei die zweite Auswerteeinheit (2, 2') dazu ausgebildet ist, anhand der Messdaten zu ermitteln, ob die vom Probennehmer aufgenommene Menge an Körperflüssigkeit einen vorbestimmten Grenzwert überschreitet; und
- ein mit der ersten Auswerteeinheit (2, 2') und der zweiten Auswerteeinheit (2a; 2a') koppelbares Steuergerät (1; 1'), das ausgestaltet ist, den Startvorgang des Fahrzeuges zu verhindern, wenn die aufgenommene Menge an Körperflüssigkeit den Grenzwert nicht überschreitet oder wenn mindestens eine der Substanzkonzentrationen oberhalb oder unterhalb eines vorbestimmten Konzentrationsgrenzwerts liegt.

2. Interlock-System nach Anspruch 1, bei dem die Körperflüssigkeit Blut, Urin, Speichel, Tränenflüssigkeit Schweiß oder interstitielle Gewebsflüssigkeit ist.

3. Interlock-System nach einem der vorhergehenden Ansprüche, bei dem die Temperatur der abgegebenen Körperflüssigkeit während der Probengabe gemessen und mit einem Sollwertbereich verglichen wird.

4. Interlock-System nach einem der vorhergehenden Ansprüche, bei dem die Messdaten ein Maß sind für einen Farbumschlag oder eine Brechungsindexänderung einer Detektionszone (24) des Probennehmers, für elektrochemische Eigenschaften der Körperflüssigkeit oder für die Leitfähigkeit der Körperflüssigkeit.

5. Interlock-System nach einem der vorhergehenden Ansprüche, bei dem die Substanzen zu einer Gruppe gehören, die illegalen Drogen wie zum Beispiel Amphetamine, Methamphetamine, Opiate, Kokain, Cannabinoide beinhaltet, oder zu einer Gruppe gehören, die therapeutische Drogen beinhaltet, wie zum Beispiel Benzodiazepine, Methadon, Buprenorphin, trizyklische Antidepressiva.

6. Interlock-System nach einem der vorhergehenden Ansprüche, bei dem die Probenahme mittels einer Kamera überwacht wird, indem optisch festgestellt wird, ob sich ein Mundstück (15) des Probennehmers (4; 4') während der Probenahme im Mund des Fahrers befindet.

7. Interlock-System nach einem der vorhergehenden Ansprüche, bei dem die Probenahme überwacht wird, indem durch biochemische Nachweisreaktion festgestellt wird, ob es sich bei der Probe um humanen Speichel beziehungsweise um den Speichel des individuellen Fahrers handelt.

8. Interlock-System nach einem der vorhergehenden Ansprüche, bei dem mit Hilfe eines Zufallsgenerators bestimmt wird, zu welchen Zeitpunkten eine Messung erfolgt.

9. Interlock-System nach einem der vorhergehenden Ansprüche, bei dem der Probennehmer (4; 4') ausgestaltet ist, um in das Steuergerät (1, 1') oder in ein mit dem Steuergerät verbundenes Handgerät (3; 3') eingesetzt zu werden.

10. Interlock-System nach einem der vorhergehenden Ansprüche, bei dem das Steuergerät (1; 1') ein mit dem Anlasser des Fahrzeugs gekoppeltes erstes Relais aufweist, das abhängig von der gemessenen Substanzkonzentration geschaltet wird und bei dem das Steuergerät (1; 1') ein zweites Relais aufweist, das parallel zum ersten Relais geschaltet ist und in Reaktion auf drahtlos empfangene Daten geschaltet werden kann.

11. Interlock-System nach einem der vorhergehenden Ansprüche, bei dem die erste Auswerteeinheit (2; 2') und/oder das Steuergerät (1; 1') einen Datenspeicher aufweist, der mit Hilfe drahtloser Datenübertragung beschrieben und ausgelesen werden kann.

12. Interlock-System nach einem der vorhergehenden Ansprüche, bei dem durch eine Anzeige auf einem Display (25, 25') und/oder durch ein akustisches Signal angezeigt wird, wenn die vom Probennehmer (4, 4') aufgenommene Menge an Körperflüssigkeit den vorbestimmten Grenzwert überschreitet.

## Claims

1. Interlock system for a vehicle comprising:
- a sampler (4, 4') which is configured to collect an adequate sample of bodily fluids from a subject to be tested;
- a readout unit (11, 11') couplable to the sampler (4, 4') for the detection of substances contained in the sample of bodily fluids;
- a first evaluation unit (2, 2') couplable to the readout unit for evaluating the substances detected by the readout unit (11, 11'), in order to obtain concentrations for each of the substances detected;
- wherein the detection and evaluation of substances in the bodily fluid takes place by means of chemical, immuno-chemical, enzymatic, electrochemical or optical detection methods, **characterized by**
- a measurement assembly (8, 8') for the automatic measurement of measurement data which are a measure of the quantity of bodily fluid that has been collected from the subject, wherein the measurement assembly (8, 8') is configured to transmit the measurement data to a second evaluation unit (2, 2') and wherein the second evaluation unit (2, 2') is configured to determine by means of the measurement data whether the quantity of bodily fluid collected from the subject exceeds a predetermined threshold value; and
- a control unit (1, 1') couplable to the first evaluation unit (2, 2') and the second evaluation unit (2a, 2a') which is configured to prevent the vehicle's starting sequence if the quantity of bodily fluid collected does not exceed the threshold value or if at least one of the substance concentrations is above or below a predetermined concentration threshold value.

2. Interlock system according to Claim 1, wherein the bodily fluid is blood, urine, saliva, lacrimal fluid, perspiration or interstitial tissue fluid.

3. Interlock system according to one of the preceding claims, wherein the temperature of the bodily fluid delivered during sampling is measured and compared with a range of target values.

4. Interlock system according to one of the preceding claims, wherein the measurement data are a measure of a colour change or a change in the refractive index of a detection zone (24) of the sampler, of electrochemical properties of the bodily fluid or of the conductivity of the bodily fluid.

5. Interlock system according to one of the preceding claims, wherein the substances belong to a group made up of illegal drugs such as amphetamines, methamphetamines, opiates, cocaine, cannabinoids, for example, or to a group made up of therapeutic drugs, such as benzodiazepines, methadone, buprenorphine, tricyclic antidepressants, for example.

6. Interlock system according to one of the preceding claims, wherein the sampling is monitored by means of a camera by visually determining whether a mouthpiece (15) of the sampler (4, 4') is located in the driver's mouth during sampling.

7. Interlock system according to one of the preceding claims, wherein the sampling is monitored by determining by means of a biochemical detection reaction whether the sample is human saliva or the saliva of the individual driver.

8. Interlock system according to one of the preceding claims, wherein a random generator is used to determine the times at which a measurement is taken.

9. Interlock system according to one of the preceding claims, wherein the sampler (4, 4') is configured to be used in the control unit (1, 1') or in a hand unit (3, 3') connected to the control unit.

10. Interlock system according to one of the preceding claims, wherein the control unit (1, 1') exhibits a first relay coupled to the vehicle starter, which relay is switched depending on the measured substance concentration and in which the control unit (1, 1') has a second relay which is switched in parallel with the first relay and can be switched in response to data received wirelessly.

11. Interlock system according to one of the preceding claims, wherein the first evaluation unit (2, 2') and/or the control unit (1, 1') has/have a data store which can be written and read with the help of wireless data transmission.

12. Interlock system according to one of the preceding claims, wherein information on a display (25, 25') and/or an acoustic signal is/are used to indicate when the quantity of bodily fluids collected by the sampler (4, 4') has exceeded the predetermined threshold value.

## Revendications

1. Système antidémarrage pour un véhicule, comprenant :
- un élément de prélèvement d'échantillon (4; 4') qui est conçu pour prélever un échantillon suffisant de fluide corporel sur une personne à tester;
- une unité de lecture (11; 11') pouvant être couplée à l'élément de prélèvement d'échantillon (4; 4') et destinée à détecter des substances qui sont contenues dans l'échantillon de fluide corporel;
- une première unité d'évaluation (2; 2') pouvant être couplée à l'unité de lecture et destinée à évaluer les substances détectées par l'unité de lecture (11; 11'), afin d'obtenir les concentrations respectives des substances détectées;
- sachant que la détection et l'évaluation des substances dans le fluide corporel sont effectuées au moyen de méthodes de dépistage chimiques, immunochimiques, enzymatiques, électrochimiques ou optiques, **caractérisé par**
- un agencement de mesure (8; 8') pour la mesure automatique de données à mesurer qui constituent une mesure de la quantité de fluide corporel recueillie par l'élément de prélèvement d'échantillon (4; 4'), l'agencement de mesure (8, 8') étant conçu pour transmettre les données mesurées à une deuxième unité d'évaluation (2, 2'), et la deuxième unité d'évaluation (2, 2') étant conçue pour déterminer à l'aide des données mesurées, si la quantité de fluide corporel recueillie par l'élément de prélèvement d'échantillon dépasse une valeur limite prédéfinie; et
- un appareil de commande (1; 1') qui peut être couplé à la première unité d'évaluation (2; 2') et à la deuxième unité d'évaluation (2a; 2a') et qui est équipé pour empêcher l'opération de démarrage du véhicule, si la quantité de fluide corporel recueillie ne dépasse pas la valeur limite ou si au moins l'une des concentrations de substance est supérieure ou inférieure à une valeur limité de concentration prédéfinie.

2. Système antidémarrage selon la revendication 1, dans lequel le fluide corporel est du sang, de l'urine, de la salive, du liquide lacrymal, de la sueur ou du liquide tissulaire.

3. Système antidémarrage selon l'une des revendications précédentes, dans lequel la température du fluide corporel délivré est mesurée pendant le prélèvement de l'échantillon et est comparée avec une plage de valeurs prescrites.

4. Système antidémarrage selon l'une des revendications précédentes, dans lequel les données mesurées constituent une mesure d'un virement de couleur ou d'un changement d'indice de réfraction d'une zone de détection (24) de l'élément de prélèvement d'échantillon (4; 4'), de propriétés électrochimiques du fluide corporel ou de la conductibilité du fluide corporel.

5. Système antidémarrage selon l'une des revendications précédentes, dans lequel les substances appartiennent à un groupe qui comprend des drogues illégales, telles que des amphétamines, les méthamphétamines, les opiacés, la cocaïne, les cannabinoïdes, ou appartiennent à un groupe comprenant des drogues thérapeutiques, telles que les benzodiazépines, la méthadone, la buprénorphine, les antidépresseurs tricycliques.

6. Système antidémarrage selon l'une des revendications précédentes, dans lequel le prélèvement de l'échantillon est surveillé au moyen d'une caméra, en déterminant par voie optique, si un embout buccal (15) de l'élément de prélèvement d'échantillon (4; 4') se trouve dans la bouche du conducteur pendant le prélèvement de l'échantillon.

7. Système antidémarrage selon l'une des revendications précédentes, dans lequel le prélèvement de l'échantillon est surveillé en déterminant par une réaction de dépistage biochimique, si l'échantillon est constitué de salive humaine, respectivement s'il s'agit de la salive du conducteur individuel.

8. Système antidémarrage selon l'une des revendications précédentes, dans lequel on définit à l'aide d'un générateur aléatoire à quels instants une mesure est effectuée.

9. Système antidémarrage selon l'une des revendications précédentes, dans lequel l'élément de prélèvement d'échantillon (4; 4') est conçu pour être inséré dans l'appareil de commande (1; 1') ou dans un appareil portatif (3; 3') relié à l'appareil de commande.

10. Système antidémarrage selon l'une des revendications précédentes, dans lequel l'appareil de commande (1; 1') présente un premier relais qui est couplé au démarreur du véhicule et qui est commuté en fonction de la concentration de substance mesurée, et dans lequel l'appareil de commande (1; 1') présente un deuxième relais qui est branché en parallèle avec le premier relais et peut être commuté en réaction à des données reçues via une transmission sans fil.

11. Système antidémarrage selon l'une des revendications précédentes, dans lequel la première unité d'évaluation (2; 2') et/ou l'appareil de commande (1; 1') présente(nt) une mémoire de données qui peut recevoir des inscriptions et être lue à l'aide d'une transmission de données sans fil/

12. Système antidémarrage selon l'une des revendications précédentes, dans lequel un affichage sur un écran (25, 25') et/ou un signal acoustique indique(nt) que la quantité de fluide corporel recueillie par l'élément de prélèvement d'échantillon (4; 4') dépasse la valeur limite prédéfinie.
